(19) Europäisches Patentamt
European Patent Office
Office européen des brevets

(11) Veröffentlichungsnummer: **0 276 741**
**A2**

(12) **EUROPÄISCHE PATENTANMELDUNG**

(21) Anmeldenummer: **88100767.8**

(51) Int. Cl.⁴: **C07C 143/68**, C07D 241/44

(22) Anmeldetag: **20.01.88**

(30) Priorität: **29.01.87 CH 319/87**

(43) Veröffentlichungstag der Anmeldung:
**03.08.88 Patentblatt 88/31**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Schnurrenberger, Klaus, Dr.**
**Liebrütistrasse 20/6**
**CH-4303 Kaiseraugst(CH)**

(74) Vertreter: **Aschert, Hubert et al**
**Grenzacherstrasse 124 Postfach 3255**
**CH-4002 Basel(CH)**

(54) **Verfahren zur Herstellung von Propionsäurederivaten.**

(57) Es wird ein neues Verfahren zur Herstellung von bekannten Isopropylidenamino-oxyäthyl-propionaten der in der Beschreibung angegebenen Formeln IV und V beschriebenen welches im wesentlichen in der Umsetzung eines Alkyl-2-[(p-tolylsulfonyl)oxy]propionats mit Isopropylidenaminooxyäthanol in Gegenwart eines Titan-oder Zirkonkatalysators besteht.

EP 0 276 741 A2

# Verfahren zur Herstellung von Propionsäurederivaten

Die vorliegende Erfindung bezieht sich auf ein neues Verfahren zur Herstellung von Isopropylidenamino-oxyäthyl-propionaten, und zwar zur Herstellung von 2-[(Isopropylidenamino)oxy]-äthyl --[(p-tolylsulfonyl)oxy]propionat der Formel

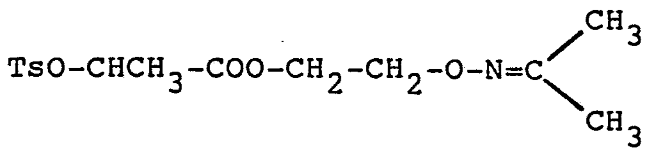

(IV)

sowie von 2-[(isopropylidenamino)oxy]äthyl-2-[p-[(6-chlor -2-chinoxalinyl)oxy]phenoxy]propionat der Formel

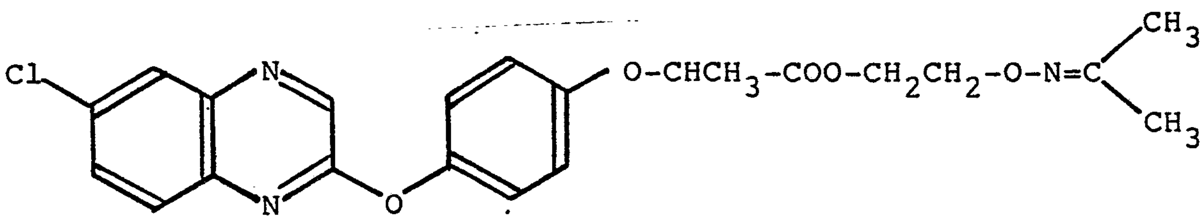

(V)

Die Verbindung der Formel IV stellt ein wichtiges Zwischenprooukt für die Herstellung der als Herbicid bekannten Verbindung der Formel V dar, in welche sie in einfacher Weise übergeführt werden kann.

Das erfindungsgemässe Verfahren ist dadurch gekennzeichnet, dass man ein Alkyl-2-[(p-tolylsulfonyl)-oxy]propionat der Formel

$TsO-CHCH_3-COOR$ (I)

worin R eine Alkylgruppe, insbesondere Aethyl bedeutet, in Gegenwart eines Titan-oder Zirkonkatalysators der allgemeinen Formel

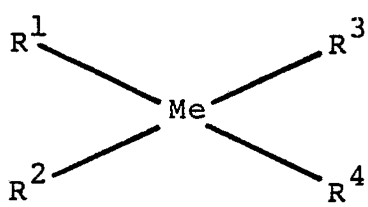

(II)

worin $R^1$, $R^2$, $R^3$ und $R^4$ gegebenenfalls substituiertes Alkoxy oder Halogen, insbesondere Chlor, bedeuten und worin Me Titan oder Zirkon darstellt,

mit Isopropylidenaminooxyäthanol der Formel

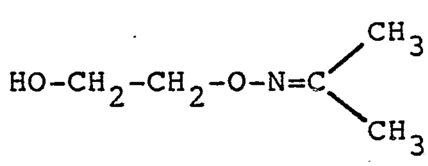

(III)

zum 2-[(Isopropylidenamino)oxy]-äthyl -[(p-tolylsulfonyl)oxy]propionat der Formel

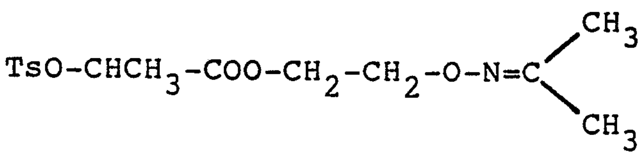

(IV)

umsetzt und dass man die erhaltene Verbindung der Formel IV gegebenenfalls in das 2-[-(isopropylidenamino)oxy]äthyl-2-[p-[(6-chlor -2-chinoxalinyl)oxy]phenoxy]propionat der Formel

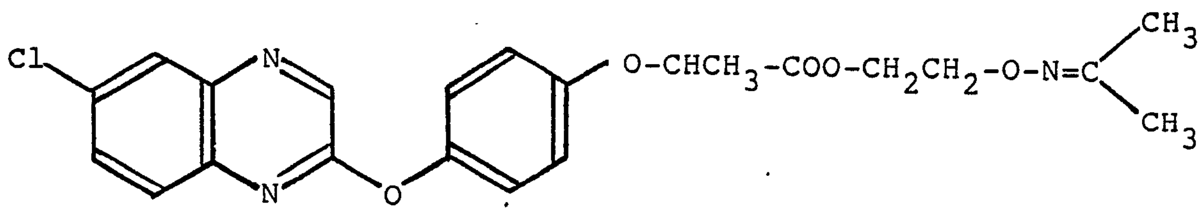

(V)

überführt.

Unter Alkyl-bzw. Alkoxygruppen sind solche mit bis etwa 20 Kohlenstoffatomen, insbesondere solche mit 1-6 Kohlenstoffatomen, vorzugsweise Aethyl, bzw. Aethoxy zu verstehen. Unter Halogen sind die vier Halogenatome zu verstehen, wobei Chlor bevorzugt ist.

Als Katalysatoren der Formel II werden insbesondere solche Verbindungen verwendet, worin die Substituenten $R^1$ bis $R^4$ jeweils eine Alkoxygruppe mit 1-3 Kohlenstoffatomen, vorzugsweise Aethoxy, bedeuten. Der besonders bevorzugte Katalysator der Formel II ist das Tetraäthyltitanat. Die Alkoxygruppen $R^1$ bis $R^4$ können auch, beispielsweise mit Hydroxy, substituiert sein; beispielsweise können $R^1$ bis $R^4$ Hydroxyäthoxygruppen oder dergleichen sein.

Der Katalysator kann in Mengen von etwa 0,1 Molprozent bis mehrere Aequivalente verwendet werden; vorzugsweise wendet man den Katalysator in einer Menge von etwa 10 Molprozent an.

Die Umsetzung der Verbindung der Formel I mit der Verbindung der Formel III kann in einem weiten Temperaturbereich, nämlich von Raumtemperatur bis weit über den Siedepunkt des verwenueten Lösungsmittels hinaus durchgeführt werden, wobei im letzteren Fall in einem Autoklaven gearbeitet wird. Vorzugsweise wird die Umsetzung bei etwa der Rück flusstemperatur des verwendeten Lösungsmittels durchgeführt.

Als Lösungsmittel können verwendet werden niedere Alkanole, wie Methanol oder Aethanol, inerte Kohlenwasserstoffe wie Benzol, Toluol, Xylol, Hexan, Pentan, Heptan oder Aethylacetat. Es können auch Zusätze, wie beispielsweise Aethylenglykol verwendet werden.

Bevorzugt wird als Ausgangsmaterial der Formel I das L-isomere verwendet, wobei man das L-isomere der Verbindung der Formel IV und das D-isomere der Formel V erhält. Das D-isomere der Formel V zeichnet sich durch eine besondere herbicide Wirkung aus.

Die allenfalls vorzunehmende Ueberführung der Verbindung der Formel IV in die Verbindung der Formel V erfolgt in bekannter Weise durch Umsetzung der Verbindung der Formel IV mit p-[(6-Chlor-2-chinoxalinyl)oxy]-phenol.

Das erfindungsgemässe Verfahren ist auch anwendbar für die Herstellung von Analogen und Homologen der Verbindungen IV und V, wie sie beispielsweise in der Europäischen Patentpublikation 52798 beschrieben sind.

Beispiel 1

In einen 2,5 1-Vierhalskolben mit Rührer, Thermometer, Rückflusskondensor, Stickstoffspülung und Oelbad werden 162 g Aethyl-L-2-[(p-tolylsulfonyl)oxy]propionat, 112 g Isopropylidenaminooxyäthanol und 1200 ml o-Xylol eingebracht.

Nach Zusatz von 15 g Tetraäthylorthotitanat wird die Mischung bei 90-100°C 2 Stunden lang gerührt, worauf der Druck auf 0,3-0,2 bar reduziert wird und der gebildete Alkohol unter weiterem Rühren bei 90-100°C abdestilliert wird. Man lässt die Reaktion während etwa 8 Stunden laufen und setzt dann 400 ml 12%ige Schwefelsäure zu, rührt während 1 Stunde und trennt die beiden Phasen.

Die organische Phase wird hierauf mit 400 ml 10%iger Schwefelsäure extrahiert.

Nach Extraktion der wässrigen Phase mit 500 ml Toluol werden die organischen Phasen vereinigt und filtriert, worauf das gesamte Lösungsmittel unter vermindertem Druck abgedampft wird. Nach Trocknung des öligen Rückstandes während 2 Stunden bei 70°C erhält man 192 g 2-[(Isopropylidenamino)oxy]äthyl -L-[(p-tolylsulfonyl)oxy]propionat.

Beispiel 2

4.4 g einer 55%igen Dispersion von Natriumhydrid in Mineralöl werden unter Rühren bei Raumtemperatur eine Lösung von 27,26 g p-[(6-chlor-2-chinoxalinyl)oxy]phenol in 100 ml absolutem Dimethylformamid zugesetzt. Man rührt während einer weiteren Stunde bei Raumtemperatur, worauf dem Reaktionsgemisch 0,26 g 1,4,7,10,13-Pentaoxycyclopentadecan und eine Lösung von 34,34 g L-2-[(Isopropylidenamino)oxy]-äthyl-2-[(p-tolylsulfonyl)oxy]propionat in 10 ml absolutem Dimethylformamid unter Rühren zugesetzt werden. Hierauf rührt man während weiteren 2 Stunden bei 60-70°C. Dem abgekühlten Reaktionsgemisch setzt man dann 1000 ml Wasser zu und extrahiert das Gemisch dreimal mit je 200 ml Aether. Die Aetherphasen werden neutral gewaschen, über Natriumsulfat getrocknet und unter vermindertem Druck zur Trockene eingedampft. Der Rückstand wird chromatographisch an einer Aluminiumoxydsäule mit Aether/Hexan (1:1) gereinigt. Nach Umkristallisation aus Aether/Hexan erhält man 2-[(Isopropylidenamino)oxy]äthyl-D-2-[p-[(6-chlor-2 -chinoxalinyl)oxy]-phenoxy]propionat mit einem Schmelzpunkt von 62-64°C;

$[\alpha]^{20}_{D}$ = +29,3° ($CHCl_3$; c = 0,10%).

## Ansprüche

1. Verfahren zur Herstellung von Isopropylidenamino-oxyäthyl-propionaten, dadurch gekennzeichnet, dass man ein Alkyl-2-[(p-tolylsulfonyl)oxy]propionat der Formel

$TsO\text{-}CHCH_3\text{-}COOR$ (I)

worin R eine Alkylgruppe, insbesondere Aethyl, bedeutet, in Gegenwart eines Titan-oder Zirkonkatalysators der allgemeinen Formel

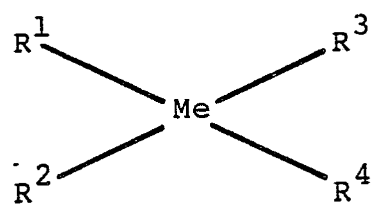

(II)

worin $R^1$, $R^2$, $R^3$ und $R^4$ gegebenenfalls substituiertes Alkoxy oder Halogen, insbesondere Chlor, bedeuten und worin Me Titan oder Zirkon darstellt,
mit Isopropylidenaminooxyäthanol der Formel

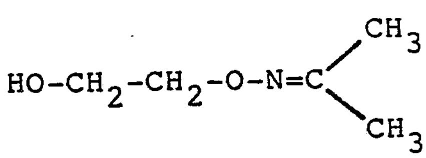

(III)

zum 2-[(isopropylidenamino)oxy]-äthyl -[(p-tolylsulfonyl)oxy]propionat der Formel

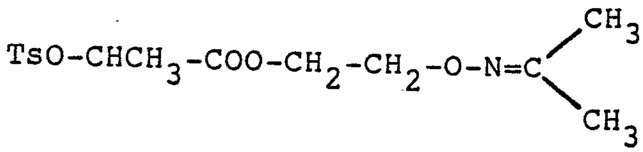

(IV)

umsetzt und die erhaltene Verbindung der Formel IV gegebenenfalls in das 2-[(Isopropylidenamino)oxy]-äthyl-2-[p-[(6-chlor -2-chinoxalinyl)oxy]phenoxy]propionat der Formel

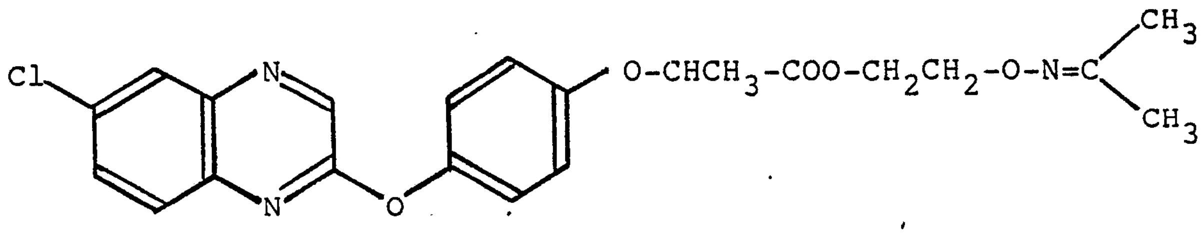

(V)

überführt.

2. Verfahren nach Anspruch 1, dadurch gekennzeichnet, dass man als Katalysator der Formel II ein Tetraalkyltitanat, insbesondere das Tetraäthyltitanat, verwendet.

3. Verfahren nach Anspruch 1 oder 2, dadurch gekennzeichnet, dass man den Katalysator in einer Menge von etwa 10 Molprozent verwendet.

4. Verfahren nach Anspruch 1, 2 oder 3, dadurch gekennzeichnet, dass man die Reaktionen in einem inerten Lösungsmittel, wie Benzol, Toluol, Xylol, Hexan, in einem niederen Alkanol oder in Aethylacetat durchführt.

5. Verfahren nach einem der Ansprüche 1-4, dadurch gekennzeichnet, dass man die Reaktion etwa bei der Rückflusstemperatur des verwendeten Lösungsmittels durchführt.

6. Verfahren nach einem der Ansprüche 1-5, dadurch gekennzeichnet, dass man als Ausgangsmaterial der Formel I das L-Isomere verwendet, wobei man das L-Isomere der Verbindung der Formel IV und das D-Isomere der Verbindung der Formel V erhält.